# FASCICULE DE BREVET EUROPEEN

(11) **EP 4 585 667 B1**
(45) Date de publication et mention de la délivrance du brevet: **19.08.2026**
(21) Numéro de dépôt: 24222122.4
(22) Date de dépôt: 20.12.2024
(51) Int. Cl.: C10L 3/10, F25J 1/00, F25J 3/02

(54) **PROCÉDÉ ET APPAREIL DE PRODUCTION DE MÉTHANE LIQUIDE**
VERFAHREN UND VORRICHTUNG ZUR HERSTELLUNG VON FLÜSSIGEM METHAN
METHOD AND APPARATUS FOR PRODUCING LIQUID METHANE

(30) Priorité: 10.01.2024 FR 2400220
(43) Date de publication de la demande: 16.07.2025
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: AYRAULT, Laura, 38360 Sassenage (FR); ROIG, Mathieu, 38360 Sassenage (FR)
(74) Mandataire: Air Liquide

(56) Documents cités:
- WO-A2-2009/004207
- WO-A2-2012/107688
- US-A1- 2014 130 542

## Description

L'invention concerne plus particulièrement un procédé de production de méthane liquide épuré en azote à partir d'un flux gazeux d'alimentation contenant au moins 95% mol de méthane et entre 0.1 et 5% mol d'azote, en particulier au moins 98 % mol de méthane et entre 0,1 et 2 % mol d'azote.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, et dont les conditions sont contrôlées, appelé méthaniseur ou digesteur, puis dans un post-digesteur, similaire au digesteur et permettant de pousser plus loin la réaction de méthanisation.

On appellera biomasse tout groupement de matières organiques pouvant se transformer en énergie à travers ce processus de méthanisation par exemple : boues de station d'épuration, fumiers/lisiers, résidus agricoles, déchets alimentaires...

Le biogaz contient majoritairement du méthane (CH4) et du dioxyde de carbone (CO2) dans des proportions variables en fonction du mode d'obtention et du substrat mais peut également contenir, en moindres proportions de l'eau, de l'azote, de l'oxygène, de l'hydrogène sulfuré (H2S) ou des composés organiques volatiles (COV).

Selon les matières organiques dégradées et les techniques utilisées, les proportions des composants diffèrent, mais en moyenne le biogaz comporte, sur gaz sec, de 30 à 75% de méthane, de 15 à 60% de CO2, jusqu'à 15% d'azote, jusqu'à 5% d'oxygène et des composés traces.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au cœur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké et transporté sous forme de gaz naturel liquide (bioGNL)...

Le procédé d'épuration « classique » (membrane ou colonne de lavage à l'eau) permet d'éliminer le CO2 jusqu'à ~1 à 2,5%. Une étape d'adsorption à température ambiante permet d'abaisser sa concentration à < 50 ppm. En revanche, les gaz de l'air ont tendance à rester avec le méthane lors des étapes d'épuration. L'air restant peut être éliminé, par exemple au moyen d'un catalyseur, jusqu'à ce qu'il en reste une faible quantité, pouvant aller jusqu'à 1 à 2 %.

Lorsqu'une très haute pureté en méthane (>99,9% par exemple), ainsi qu'un bon taux de récupération (>99%) sont recherchés, ces briques d'épuration ne permettent pas d'atteindre ces objectifs. La présente invention décrit une solution pour liquéfier le biométhane et l'épurer en azote avec la même installation. L'invention peut également être appliquée à la liquéfaction de toute source de méthane contenant de l'azote, par exemple le gaz naturel ou un mélange de gaz naturel et de biométhane.

Le document publié sous le numéro EP 3465035 A1 décrit un procédé de distillation cryogénique comme étape d'épuration de biogaz contenant des quantités d'air (azote et oxygène) relativement hautes (entre 3 et 50% molaire d'azote et d'oxygène). Les concentrations du méthane visées en sortie sont celles compatibles avec les spécifications pour réinjection dans le réseau de gaz naturel, ou pour le carburant véhicule ce qui correspond à CH4mol > 97,5%. Il propose en particulier, par la conception de la distillation cryogénique, de résoudre le problème d'atmosphères explosives qui pourraient être créées avec une distillation classique, lorsqu'on doit épurer un biogaz riche en gaz de l'air.

Les documents publiés sous les numéros FR 2971331 A1 et FR 2971332 A1 proposent également des procédés de distillation cryogénique pour la purification du biogaz contenant entre 65 et 97% de méthane, le reste étant de l'air. Pour éviter la formation d'un mélange explosif dans la colonne, les mélanges gazeux et/ou liquides de la colonne de distillation sont dilués par réinjection d'une partie du méthane liquide en cuve de colonne ou par injection d'azote gazeux d'une source extérieure dans la cuve.

L'art antérieur décrit ci-dessus propose des solutions de distillation cryogénique comme étape d'épuration de biogaz contenant des quantités d'air (azote et oxygène) relativement élevées afin d'éviter le risque d'explosion à l'intérieur d'une colonne de distillation. Cependant, ces documents ne concernent pas la liquéfaction du méthane en tant que tel, notamment avec l'élimination de l'azote résiduel, ou l'optimisation d'une conception pour ce besoin.

A cette fin, le procédé selon l'invention, par ailleurs conforme à la définition générique qu'en donne le préambule ci-dessus, est essentiellement caractérisée en ce que :
- le flux gazeux d'alimentation est refroidi à une température comprise entre 110 K et 200 K, en particulier entre 130 K et 150 K, dans un premier échangeur de chaleur à contre-courant d'un flux d'azote vapeur pour produire un flux refroidi,
- un bain d'azote liquide est en échange thermique avec le flux refroidi via un deuxième échangeur de chaleur dans lequel transite le flux refroidi, le bain d'azote liquide produisant de l'azote vapeur fourni au premier échangeur de chaleur pour produire le flux refroidi,
- le flux refroidi sortant du deuxième échangeur de chaleur est introduit dans une colonne de distillation en vue de sa distillation, et
- un liquide contenant au moins 99,5 % mol, de préférence au moins 99,9 % mol, de méthane est soutiré en cuve de la colonne de distillation comme produit final.

Par ailleurs, des modes de réalisation de l'invention peuvent comporter l'une ou plusieurs des caractéristiques suivantes :
- Le flux gazeux d'alimentation contient au moins 99% mol de méthane, en particulier au moins 99,5% mol de méthane et entre 0,1 et 1% mol d'azote, en particulier entre 0,1 et 0,5%mol d'azote.
- Avant l'introduction dans la colonne de distillation, le flux refroidi sortant du deuxième échangeur de chaleur transite dans un bouilleur de la colonne.
- Une phase vapeur appauvrie en méthane et enrichie en azote par rapport au flux gazeux d'alimentation est soutirée en tête de la colonne de distillation et envoyée à un condenseur dans lequel la phase vapeur est refroidie par échange de chaleur avec l'azote liquide afin de condenser une partie de la phase vapeur formant un gaz enrichi en azote par rapport à la phase vapeur et un liquide enrichi en méthane par rapport à la phase vapeur.
- Le liquide enrichi en méthane est renvoyé à la colonne de distillation.
- L'azote liquide est vaporisé dans le condenseur formant l'azote vapeur qui est fourni au premier échangeur de chaleur pour produire le flux refroidi.
- Le condenseur comprend un échangeur de chaleur à plaques baigné dans l'azote liquide.
- Le liquide soutiré en cuve de la colonne de distillation est refroidi dans un troisième échangeur de chaleur par échange de chaleur avec l'azote liquide, de l'azote liquide étant vaporisé formant l'azote vapeur qui est fourni au premier échangeur de chaleur pour produire le flux refroidi.
- Le flux refroidi est introduit en partie supérieure de la colonne de distillation.

L'invention concerne également un appareil de production de méthane liquide épuré en azote à partir d'un flux gazeux d'alimentation contenant au moins 95 % mol de méthane et entre 0,1 et 5 % mol d'azote, en particulier au moins 98% mol de méthane et entre 0.1 et 2% mol de l'azote, l'installation comprenant :
- un premier échangeur de chaleur configuré pour refroidir le flux gazeux d'alimentation à une température comprise entre 110 K et 200 K, en particulier entre 130 K et 150 K, à contre-courant d'un flux d'azote vapeur pour produire d'un flux refroidi,
- une deuxième échangeur de chaleur plongé dans un bain d'azote liquide configuré pour vaporiser l'azote liquide par échange de chaleur avec le flux refroidi pour produire de l'azote vapeur,
- une colonne de distillation configuré pour distiller le flux refroidi et produire un liquide contenant au moins 99,5 % mol, de préférence au moins 99,9 % mol de méthane en cuve de la colonne,
- un bouilleur de la colonne configuré pour recevoir le flux refroidi avant son introduction dans la colonne de distillation,
- un condenseur comprenant un échangeur de chaleur à plaques baigné dans l'azote liquide, le condenseur étant configuré pour refroidir une phase vapeur soutirée en tête de la colonne de distillation par échange de chaleur avec l'azote liquide, pour former un gaz enrichi en azote par rapport à la phase vapeur et un liquide enrichi en méthane par rapport à la phase vapeur, et pour produire de l'azote vapeur,
- au moins un premier conduit configuré pour envoyer le flux refroidi sortant du premier échangeur de chaleur au deuxième échangeur de chaleur,
- au moins un deuxième conduit configuré pour envoyer l'azote vapeur sortant du deuxième échangeur de chaleur au premier échangeur de chaleur,
- au moins un troisième conduit configuré pour envoyer le flux refroidi sortant du deuxième échangeur de chaleur au bouilleur,
- au moins un quatrième conduit configuré pour envoyer le flux refroidi sortant du bouilleur à la colonne de distillation,
- au moins un cinquième conduit configuré pour soutirer en tête de la colonne de distillation la phase vapeur et pour l'envoyer au condenseur,
- au moins un sixième conduit configuré pour renvoyer le liquide enrichi en méthane formé dans le condenseur à la colonne de distillation,
- au moins un septième conduit configuré pour envoyer l'azote vapeur sortant du condenseur au premier échangeur de chaleur, et
- au moins un huitième conduit configuré pour soutirer le liquide contenant au moins 99,5 % mol, de préférence au moins 99,9 % mol de méthane en cuve de la colonne comme produit final.

Selon d'autres particularités possibles :
- L'appareil comprend un troisième échangeur de chaleur plongé dans un bain d'azote liquide configuré pour refroidir le liquide soutiré en cuve de la colonne de distillation et vaporiser l'azote liquide pour produire l'azote vapeur, et moins un neuvième conduit configuré pour envoyer l'azote vapeur produit au premier échangeur de chaleur.
- L'appareil comprend un stockage de l'azote liquide relié au deuxième échangeur de chaleur et/ou le troisième échangeur de chaleur et/ou le condenseur.

La présente invention propose une solution permettant de liquéfier d'un flux riche en méthane, en particulier issue d'une épuration de biogaz et/ou le gaz naturel, et contenant de l'azote, en particulier une teneur en azote résiduelle, et de l'épurer en azote de façon efficace dans une seule installation. Il est possible de liquéfier et épurer un flux gazeux contenant au moins 95% mol, en particulier au moins 99,5% mol, de méthane et 5% ou moins d'azote, en particulier 0,5% mol ou moins d'azote par distillation cryogénique. La création d'atmosphère explosive due à l'oxygène n'est pas une préoccupation.

L'invention peut concerner également tout dispositif ou procédé alternatif comprenant toute combinaison des caractéristiques ci-dessus ou ci-dessous dans le cadre des revendications. D'autres particularités et avantages apparaîtront à la lecture de la description ci-après, faite en référence à la figure.

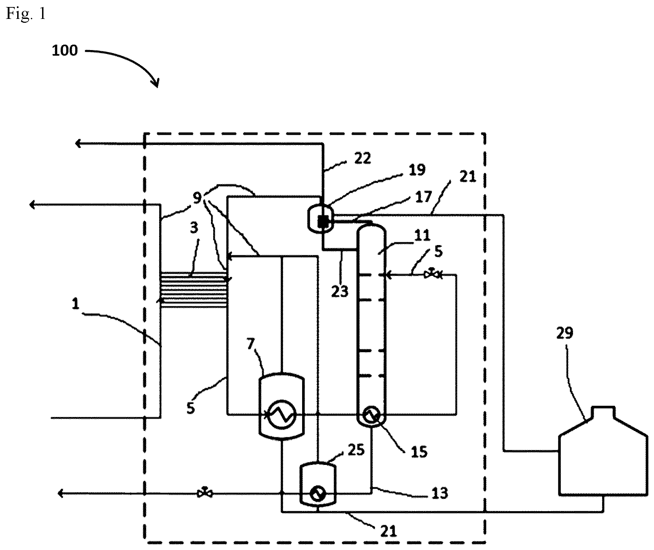

Fig. 1 illustre un exemple d'un appareil et d'un procédé selon l'invention de manière schématique.

Un flux gazeux 1 est alimenté dans l'appareil 100 pour produire du méthane liquide 13 épuré en azote. Le flux gazeux 1 d'alimentation contient au moins 95 % mol de méthane et jusqu'à 5 % mol d'azote. En particulier, le flux gazeux 1 d'alimentation contient au moins 98% mol de méthane et entre 0,1 et 2% mol d'azote. Plus particulièrement, le flux gazeux 1 d'alimentation contient au moins 99% mol de méthane et entre 0,1 et 1% mol d'azote. Encore plus particulièrement, le flux gazeux 1 d'alimentation contient au moins 99,5% mol de méthane et entre 0,1 et 0,5% mol d'azote.

Le flux gazeux 1 d'alimentation est par exemple du biométhane produit à partir de l'épuration de biogaz, par exemple par séparation membranaire, par colonne de lavage par exemple à l'eau ou au solvant, par distillation cryogénique et/ou par adsorption par exemple à température ambiante ou à température et/ou pression modulée.

Le flux gazeux 1 d'alimentation peut être du gaz naturel ou un mélange du gaz naturel et du biométhane.

Le flux gazeux 1 d'alimentation est refroidi dans un premier échangeur 3 de chaleur à contre-courant d'un flux d'azote vapeur 9. Le premier échangeur 3 de chaleur est par exemple du type échangeur à plaques brassées aluminium ("Brazed Aluminium Heat Exchangers" en anglais ou BAHX).

Le flux refroidi 5 à l'issue du premier échangeur 3 de chaleur est à une température comprise entre 110 K et 200 K, en particulier entre 130 K et 150 K.

Le flux refroidi 5 sortant du premier échangeur 3 de chaleur transite dans un deuxième échangeur 7 de chaleur qui est par exemple immergé dans un bain d'azote liquide, dans lequel le flux refroidi 5 et le bain d'azote liquide sont en échange thermique. Le flux refroidi 5 réchauffe le bain d'azote liquide du deuxième échangeur 7 de chaleur par échange thermique. Cette étape abaisse la température du flux gazeux refroidi 5 d'environ 1K, permettant ainsi de vaporiser une partie d'azote liquide pour l'intégrer sous forme vapeur dans le premier échangeur 3 de chaleur, tout en valorisant la chaleur latente de vaporisation. L'azote vapeur 9 produit est renvoyé au premier échangeur 3 de chaleur pour refroidir le flux gazeux 1 d'alimentation par échange thermique.

L'utilisation d'un bain d'azote permet d'éviter les échangeurs diphasiques ainsi que le contact direct entre l'azote liquide et le flux gazeux 5.

Le niveau du bain est maintenu constant par ajout d'azote liquide depuis la source 29, afin de compenser la perte de niveau par vaporisation.

Le flux refroidi 5 sortant du deuxième échangeur 7 de chaleur est introduit dans une colonne de distillation 11 en vue de sa distillation. Le flux refroidi 5 peut être introduit dans une zone de la colonne 11 de distillation selon notamment sa composition et les conditions opératoires visées, en particulier en partie supérieure de la colonne 11 de distillation.

De préférence, avant l'introduction dans la colonne de distillation 11, le flux refroidi 5 sortant du deuxième échangeur 7 de chaleur transite dans un bouilleur 15 de la colonne. Le flux refroidi 5 récupère de puissance froide pour finir le refroidissement, tout en délivrant une puissance chaude au bouilleur 15, par échange de chaleur dans le bouilleur 15. Le bouilleur 15 de la colonne peut se trouver en cuve de la colonne 11 ou à l'extérieur de la colonne 11. Comme illustré, une phase vapeur 17 appauvrie en méthane et enrichie en azote par rapport au flux gazeux d'alimentation 1, 5 est soutirée en tête de la colonne 11 de distillation. Cette phase vapeur 17 peut être envoyée à un condenseur 19, qui est par exemple distinct de la colonne de distillation 11. Le condenseur 19 peut comprendre ou peut être composé d'un échangeur à plaques baigné dans de l'azote liquide 21. La phase vapeur 17 est refroidie par échange de chaleur avec l'azote liquide 21, de préférence dans un bain d'azote liquide 21, dans le condenseur 19 afin de condenser une partie de la phase vapeur 17, formant un liquide 23 enrichi en méthane et appauvri en azote par rapport à la phase vapeur 17 et un gaz 22 enrichi en azote et appauvri en méthane par rapport à la phase vapeur 17.

Le liquide 23 enrichi en méthane est envoyé en tête de la colonne 11 pour constituer un liquide de reflux. Le gaz 22 enrichi en azote est évacué vers une ligne d'évent, une valorisation du méthane résiduel et/ou de la puissance froide résiduelle peut être envisagée.

Dans le condenseur 19, l'azote liquide 21 est vaporisée 9 et réintégrée dans le premier échangeur 3 de chaleur pour refroidir le flux gazeux d'alimentation 1 par échange thermique.

Un liquide 13 contenant au moins 99,5 % mol, de préférence au moins 99,9 % mol, voire au moins 99,99 % mol de méthane est soutiré en cuve ou en bas de la colonne de distillation 11 comme produit final. Le liquide 13 soutiré peut être refroidi dans un troisième échangeur 25 de chaleur par échange de chaleur avec l'azote liquide 21, en particulier un bain d'azote liquide. Le troisième échangeur 25 de chaleur est de préférence un échangeur à plaques baigné dans un bain d'azote liquide. Le liquide 13 est en particulier à l'état liquide saturé à une température d'environ 130 K. Sa température est abaissée à environ 110 K via le troisième échangeur 25 de chaleur. Le refroidissement dans le troisième échangeur 25 permet de prévenir la vaporisation du méthane liquéfié. L'azote liquide 21 ainsi vaporisée 9 peut être revalorisée dans le premier échangeur 3 de chaleur pour refroidir le flux gazeux d'alimentation 1 par échange thermique.

Le procédé selon l'invention, en particulier le refroidissement des flux et la distillation, est de préférence réalisé dans une boîte froide (cadre pointillé dans la figure). L'appareil 100, en particulier les échangeurs de chaleur 3, 7, 19, 25 et la colonne de distillation 11, est de préférence maintenu dans la boîte froide.

L'azote liquide 21 utilisé dans le procédé selon l'invention est de préférence pressurisé afin d'éviter une température qui engendrerait le gel du méthane.

Le procédé selon l'invention permet de mutualiser la source réfrigérant pour le condenseur, le (pré-)refroidissement et la liquéfaction, et d'intégrer le flux de réfrigérant (l'azote) vapeur dans un même échangeur de (pré-)refroidissement (le premier échangeur 3 de chaleur).

Il est possible d'atteindre un taux de récupération de la molécule de méthane supérieur à 99,9 %, tout en minimisant la consommation de réfrigérant.

## Revendications

1. Procédé de production de méthane liquide épuré en azote à partir d'un flux gazeux d'alimentation (1) contenant au moins 95 % mol de méthane et entre 0,1 et 5 % mol d'azote, en particulier au moins 98 % mol de méthane et entre 0,1 et 2 % mol d'azote, dans lequel :
- le flux gazeux d'alimentation (1) est refroidi à une température comprise entre 110 K et 200 K, en particulier entre 130 K et 150 K, dans un premier échangeur (3) de chaleur à contre-courant d'un flux d'azote vapeur (9) pour produire un flux refroidi (5),
- un bain d'azote liquide (21) est en échange thermique avec le flux refroidi (5) via un deuxième échangeur (7) de chaleur dans lequel transite le flux refroidi (5), le bain d'azote liquide (21) produisant de l'azote vapeur (9) fourni au premier échangeur (3) de chaleur pour produire le flux refroidi,
- le flux refroidi (5) sortant du deuxième échangeur (7) de chaleur est introduit dans une colonne de distillation (11) en vue de sa distillation, et
- un liquide (13) contenant au moins 99,5 % mol, de préférence au moins 99,9 % mol, de méthane est soutiré en cuve de la colonne de distillation (11) comme produit final.

2. Procédé selon la revendication 1 dans lequel, avant l'introduction dans la colonne de distillation (11), le flux refroidi (5) sortant du deuxième échangeur (7) de chaleur transite dans un bouilleur (15) de la colonne.

3. Procédé selon la revendication 1 ou 2 dans lequel une phase vapeur (17) appauvrie en méthane et enrichie en azote par rapport au flux gazeux d'alimentation est soutirée en tête de la colonne de distillation et envoyée à un condenseur (19) dans lequel la phase vapeur (17) est refroidie par échange de chaleur avec l'azote liquide (21) afin de condenser une partie de la phase vapeur (17) formant un gaz (22) enrichi en azote par rapport à la phase vapeur (17) et un liquide (23) enrichi en méthane par rapport à la phase vapeur (17).

4. Procédé selon la revendication 3 dans lequel le liquide (23) enrichi en méthane est renvoyé à la colonne de distillation.

5. Procédé selon la revendication 3 ou 4 dans lequel l'azote liquide (21) est vaporisé dans le condenseur (19) formant l'azote vapeur (9) qui est fourni au premier échangeur (3) de chaleur pour produire le flux refroidi (5).

6. Procédé selon l'une quelconque des revendications 3 à 5 dans lequel le condenseur (19) comprend un échangeur de chaleur à plaques baigné dans l'azote liquide.

7. Procédé selon l'une quelconque des revendications 1 à 6 dans lequel le liquide (13) soutiré en cuve de la colonne (11) de distillation est refroidi dans un troisième échangeur (25) de chaleur par échange de chaleur avec l'azote liquide (21), de l'azote liquide étant vaporisé formant l'azote vapeur (9) qui est fourni au premier échangeur (3) de chaleur pour produire le flux refroidi.

8. Procédé selon l'une quelconque des revendications 1 à 7 dans lequel le flux refroidi (5) est introduit en partie supérieure de la colonne de distillation (11).

9. Appareil (100) de production de méthane liquide épuré en azote à partir d'un flux gazeux d'alimentation (1) contenant au moins 95 % mol de méthane et entre 0,1 et 5 % mol d'azote, en particulier au moins 98% mol de méthane et entre 0.1 et 2% mol de l'azote, l'installation comprenant :
- un premier échangeur (3) de chaleur configuré pour refroidir le flux gazeux d'alimentation (1) à une température comprise entre 110 K et 200 K, en particulier entre 130 K et 150 K, à contre-courant d'un flux d'azote vapeur (9) pour produire d'un flux refroidi (5),
- une deuxième échangeur (7) de chaleur plongé dans un bain d'azote liquide (21) configuré pour vaporiser l'azote liquide (21) par échange de chaleur avec le flux refroidi (5) pour produire de l'azote vapeur (9),
- une colonne de distillation (11) configuré pour distiller le flux refroidi (5) et produire un liquide (13) contenant au moins 99,5 % mol, de préférence au moins 99,9 % mol de méthane en cuve de la colonne (11),
- un bouilleur (15) de la colonne configuré pour recevoir le flux refroidi (5) avant son introduction dans la colonne (11) de distillation,
- un condenseur (19) comprenant un échangeur de chaleur à plaques baigné dans l'azote liquide (21), le condenseur étant configuré pour refroidir une phase vapeur (17) soutirée en tête de la colonne de distillation par échange de chaleur avec l'azote liquide (21), pour former un gaz (22) enrichi en azote par rapport à la phase vapeur (17) et un liquide (23) enrichi en méthane par rapport à la phase vapeur (17), et pour produire de l'azote vapeur (9),
- au moins un premier conduit configuré pour envoyer le flux refroidi (5) sortant du premier échangeur (3) de chaleur au deuxième échangeur (7) de chaleur,
- au moins un deuxième conduit configuré pour envoyer l'azote vapeur (9) sortant du deuxième échangeur (7) de chaleur au premier échangeur (3) de chaleur,
- au moins un troisième conduit configuré pour envoyer le flux refroidi (5) sortant du deuxième échangeur (7) de chaleur au bouilleur (15),
- au moins un quatrième conduit configuré pour envoyer le flux refroidi (5) sortant du bouilleur (15) à la colonne de distillation (11),
- au moins un cinquième conduit configuré pour soutirer en tête de la colonne de distillation la phase vapeur (17) et pour l'envoyer au condenseur (19),
- au moins un sixième conduit configuré pour renvoyer le liquide (23) enrichi en méthane formé dans le condenseur (19) à la colonne de distillation (11),
- au moins un septième conduit configuré pour envoyer l'azote vapeur (9) sortant du condenseur (19) au premier échangeur (3) de chaleur, et
- au moins un huitième conduit configuré pour soutirer le liquide (13) contenant au moins 99,5 % mol, de préférence au moins 99,9 % mol de méthane en cuve de la colonne comme produit final.

10. Appareil (100) selon la revendication 9 comprenant un troisième (25) échangeur de chaleur plongé dans un bain d'azote liquide (21) configuré pour refroidir le liquide (13) soutiré en cuve de la colonne de distillation et vaporiser l'azote liquide (21) pour produire l'azote vapeur (9), et au moins un neuvième conduit configuré pour envoyer l'azote vapeur (9) produit au premier échangeur (3) de chaleur.

11. Appareil (100) selon la revendication 9 ou 10 comprenant un stockage (29) de l'azote liquide relié au deuxième échangeur (7) de chaleur et/ou le troisième échangeur (25) de chaleur et/ou le condenseur (19).

## Patentansprüche

1. Verfahren zur Herstellung von von Stickstoff gereinigtem flüssigem Methan aus einem gasförmigen Zufuhrstrom (1), der mindestens 95 Mol-% Methan und zwischen 0,1 und 5 Mol-% Stickstoff, insbesondere mindestens 98 Mol-% Methan und zwischen 0,1 und 2 Mol-% Stickstoff enthält, wobei:
- der gasförmige Zufuhrstrom (1) in einem ersten Wärmetauscher (3) im Gegenstrom zu einem Stickstoffdampfstrom (9) auf eine Temperatur zwischen 110 K und 200 K, insbesondere zwischen 130 K und 150 K, gekühlt wird, um einen gekühlten Strom (5) zu erzeugen,
- ein Bad aus flüssigem Stickstoff (21) in Wärmeaustausch mit dem gekühlten Strom (5) über einen zweiten Wärmetauscher (7) steht, durch den der gekühlte Strom (5) strömt, wobei das Bad aus flüssigem Stickstoff (21) Stickstoffdampf (9) erzeugt, der dem ersten Wärmetauscher (3) zugeführt wird, um den gekühlten Strom zu erzeugen,
- der aus dem zweiten Wärmetauscher (7) austretende gekühlte Strom (5) in eine Destillationskolonne (11) zu deren Destillation eingeleitet wird, und
- eine Flüssigkeit (13), die mindestens 99,5 Mol-%, vorzugsweise mindestens 99,9 Mol-%, Methan enthält, als Endprodukt aus dem Sumpf der Destillationskolonne (11) entnommen wird.

2. Verfahren nach Anspruch 1, wobei vor der Einleitung in die Destillationskolonne (11) der aus dem zweiten Wärmetauscher (7) austretende gekühlte Strom (5) durch einen Aufkocher (15) der Kolonne geleitet wird.

3. Verfahren nach Anspruch 1 oder 2, wobei eine an Methan verarmte und im Verhältnis zum gasförmigen Zufuhrstrom an Stickstoff angereicherte Dampfphase (17) am Kopf der Destillationskolonne entnommen und einem Kondensator (19) zugeführt wird, in dem die Dampfphase (17) durch Wärmeaustausch mit flüssigem Stickstoff (21) gekühlt wird, um einen Teil der Dampfphase (17) zu kondensieren, wodurch ein Gas (22), das im Verhältnis zur Dampfphase (17) an Stickstoff angereichert ist, und eine Flüssigkeit (23), die im Verhältnis zur Dampfphase (17) an Methan angereichert ist, gebildet werden.

4. Verfahren nach Anspruch 3, wobei die mit Methan angereicherte Flüssigkeit (23) in die Destillationskolonne zurückgeführt wird.

5. Verfahren nach Anspruch 3 oder 4, wobei der flüssige Stickstoff (21) im Kondensator (19) verdampft wird, wodurch der Stickstoffdampf (9) gebildet wird, der dem ersten Wärmetauscher (3) zugeführt wird, um den gekühlten Strom (5) zu erzeugen.

6. Verfahren nach einem der Ansprüche 3 bis 5, wobei der Kondensator (19) einen Plattenwärmetauscher umfasst, der in den flüssigen Stickstoff eingetaucht ist.

7. Verfahren nach einem der Ansprüche 1 bis 6, wobei die aus dem Sumpf der Destillationskolonne (11) entnommene Flüssigkeit (13) in einem dritten Wärmetauscher (25) durch Wärmeaustausch mit dem flüssigen Stickstoff (21) gekühlt wird, wobei der flüssige Stickstoff verdampft wird, wodurch der Stickstoffdampf (9) gebildet wird, der dem ersten Wärmetauscher (3) zugeführt wird, um den gekühlten Strom zu erzeugen.

8. Verfahren nach einem der Ansprüche 1 bis 7, wobei der gekühlte Strom (5) in einen oberen Teil der Destillationskolonne (11) eingeleitet wird.

9. Vorrichtung (100) zur Herstellung von von Stickstoff gereinigtem flüssigem Methan aus einem gasförmigen Zufuhrstrom (1), der mindestens 95 Mol-% Methan und zwischen 0,1 und 5 Mol-% Stickstoff, insbesondere mindestens 98 Mol-% Methan und zwischen 0,1 und 2 Mol-% Stickstoff enthält, wobei die Anlage umfasst:
- einen ersten Wärmetauscher (3), der so konfiguriert ist, dass er den gasförmigen Zufuhrstrom (1) im Gegenstrom zu einem Stickstoffdampfstrom (9) auf eine Temperatur zwischen 110 K und 200 K, insbesondere zwischen 130 K und 150 K, kühlt, um einen gekühlten Strom (5) zu erzeugen,
- einen zweiten Wärmetauscher (7), der in ein Bad aus flüssigem Stickstoff (21) eingetaucht ist und so konfiguriert ist, dass er den flüssigen Stickstoff (21) durch Wärmeaustausch mit dem gekühlten Strom (5) verdampft, um Stickstoffdampf (9) zu erzeugen,
- eine Destillationskolonne (11), die so konfiguriert ist, dass sie den gekühlten Strom (5) destilliert und eine Flüssigkeit (13) erzeugt, die mindestens 99,5 Mol-%, vorzugsweise mindestens 99,9 Mol-%, Methan enthält, im Sumpf der Kolonne (11),
- einen Aufkocher (15) der Kolonne, der so konfiguriert ist, dass er den gekühlten Strom (5) vor seiner Einleitung in die Destillationskolonne (11) aufnimmt,
- einen Kondensator (19), der einen in flüssigen Stickstoff (21) eingetauchten Plattenwärmetauscher umfasst, wobei der Kondensator so konfiguriert ist, dass er eine am Kopf der Destillationskolonne entnommene Dampfphase (17) durch Wärmeaustausch mit dem flüssigen Stickstoff (21) kühlt, um ein Gas (22), das im Verhältnis zur Dampfphase (17) an Stickstoff angereichert ist, und eine Flüssigkeit (23), die im Verhältnis zur Dampfphase (17) an Methan angereichert ist, zu bilden, und um Stickstoffdampf (9) zu erzeugen,
- mindestens eine erste Leitung, die so konfiguriert ist, dass sie den aus dem ersten Wärmetauscher (3) austretenden gekühlten Strom (5) zum zweiten Wärmetauscher (7) leitet,
- mindestens eine zweite Leitung, die so konfiguriert ist, dass sie den aus dem zweiten Wärmetauscher (7) austretenden Stickstoffdampf (9) zum ersten Wärmetauscher (3) leitet,
- mindestens eine dritte Leitung, die so konfiguriert ist, dass sie den aus dem zweiten Wärmetauscher (7) austretenden gekühlten Strom (5) zum Aufkocher (15) leitet,
- mindestens eine vierte Leitung, die so konfiguriert ist, dass sie den aus dem Aufkocher (15) austretenden gekühlten Strom (5) zur Destillationskolonne (11) leitet,
- mindestens eine fünfte Leitung, die so konfiguriert ist, dass sie die Dampfphase (17) am Kopf der Destillationskolonne entnimmt und sie zum Kondensator (19) leitet,
- mindestens eine sechste Leitung, die so konfiguriert ist, dass sie die im Kondensator (19) gebildete, mit Methan angereicherte Flüssigkeit (23) zur Destillationskolonne (11) zurückführt,
- mindestens eine siebte Leitung, die so konfiguriert ist, dass sie den aus dem Kondensator (19) austretenden Stickstoffdampf (9) zum ersten Wärmetauscher (3) leitet, und
- mindestens eine achte Leitung, die so konfiguriert ist, dass sie die Flüssigkeit (13), die mindestens 99,5 Mol-%, vorzugsweise mindestens 99,9 Mol-%, Methan enthält, als Endprodukt aus dem Sumpf der Kolonne entnimmt.

10. Vorrichtung (100) nach Anspruch 9, umfassend einen dritten (25) Wärmetauscher, der in ein Bad aus flüssigem Stickstoff (21) eingetaucht ist und konfiguriert ist, um die aus dem Sumpf der Destillationskolonne entnommene Flüssigkeit (13) zu kühlen und den flüssigen Stickstoff (21) zu verdampfen, um den Stickstoffdampf (9) zu erzeugen, und mindestens eine neunte Leitung, die konfiguriert ist, um den erzeugten Stickstoffdampf (9) zum ersten Wärmetauscher (3) zu leiten.

11. Vorrichtung (100) nach Anspruch 9 oder 10, umfassend einen Speicher (29) für flüssigen Stickstoff, der mit dem zweiten Wärmetauscher (7) und/oder dem dritten Wärmetauscher (25) und/oder dem Kondensator (19) verbunden ist.

## Claims

1. A method for producing liquid methane purified of nitrogen from a gaseous feed stream (1) containing at least 95 mol% methane and between 0.1 and 5 mol% nitrogen, in particular at least 98 mol% methane and between 0.1 and 2 mol% nitrogen, wherein:
- the gaseous feed stream (1) is cooled to a temperature between 110 K and 200 K, in particular between 130 K and 150 K, in a first heat exchanger (3) counter-current to a stream of nitrogen vapor (9) to produce a cooled stream (5),
- a bath of liquid nitrogen (21) is in thermal exchange with the cooled stream (5) via a second heat exchanger (7) through which the cooled stream (5) passes, the bath of liquid nitrogen (21) producing nitrogen vapor (9) supplied to the first heat exchanger (3) to produce the cooled stream,
- the cooled stream (5) exiting the second heat exchanger (7) is introduced into a distillation column (11) for its distillation, and
- a liquid (13) containing at least 99.5 mol%, preferably at least 99.9 mol%, of methane is withdrawn from the bottom of the distillation column (11) as a final product.

2. The method according to claim 1, wherein, before introduction into the distillation column (11), the cooled stream (5) exiting the second heat exchanger (7) passes through a reboiler (15) of the column.

3. The method according to claim 1 or 2, wherein a vapor phase (17) depleted in methane and enriched in nitrogen relative to the gaseous feed stream is withdrawn from the top of the distillation column and sent to a condenser (19) in which the vapor phase (17) is cooled by heat exchange with liquid nitrogen (21) in order to condense a part of the vapor phase (17) forming a gas (22) enriched in nitrogen relative to the vapor phase (17) and a liquid (23) enriched in methane relative to the vapor phase (17).

4. The method according to claim 3, wherein the liquid (23) enriched in methane is returned to the distillation column.

5. The method according to claim 3 or 4, wherein the liquid nitrogen (21) is vaporized in the condenser (19) forming the nitrogen vapor (9) which is supplied to the first heat exchanger (3) to produce the cooled stream (5).

6. The method according to any one of claims 3 to 5, wherein the condenser (19) comprises a plate heat exchanger immersed in the liquid nitrogen.

7. The method according to any one of claims 1 to 6, wherein the liquid (13) withdrawn from the bottom of the distillation column (11) is cooled in a third heat exchanger (25) by heat exchange with the liquid nitrogen (21), the liquid nitrogen being vaporized forming the nitrogen vapor (9) which is supplied to the first heat exchanger (3) to produce the cooled stream.

8. The method according to any one of claims 1 to 7, wherein the cooled stream (5) is introduced into an upper part of the distillation column (11).

9. An apparatus (100) for producing liquid methane purified of nitrogen from a gaseous feed stream (1) containing at least 95 mol% methane and between 0.1 and 5 mol% nitrogen, in particular at least 98 mol% methane and between 0.1 and 2 mol% nitrogen, the installation comprising:
- a first heat exchanger (3) configured to cool the gaseous feed stream (1) to a temperature between 110 K and 200 K, in particular between 130 K and 150 K, counter-current to a stream of nitrogen vapor (9) to produce a cooled stream (5),
- a second heat exchanger (7) immersed in a bath of liquid nitrogen (21) configured to vaporize the liquid nitrogen (21) by heat exchange with the cooled stream (5) to produce nitrogen vapor (9),
- a distillation column (11) configured to distill the cooled stream (5) and to produce a liquid (13) containing at least 99.5 mol%, preferably at least 99.9 mol%, of methane at the bottom of the column (11),
- a reboiler (15) of the column configured to receive the cooled stream (5) before its introduction into the distillation column (11),
- a condenser (19) comprising a plate heat exchanger immersed in liquid nitrogen (21), the condenser being configured to cool a vapor phase (17) withdrawn from the top of the distillation column by heat exchange with the liquid nitrogen (21), to form a gas (22) enriched in nitrogen relative to the vapor phase (17) and a liquid (23) enriched in methane relative to the vapor phase (17), and to produce nitrogen vapor (9),
- at least a first conduit configured to send the cooled stream (5) exiting the first heat exchanger (3) to the second heat exchanger (7),
- at least a second conduit configured to send the nitrogen vapor (9) exiting the second heat exchanger (7) to the first heat exchanger (3),
- at least a third conduit configured to send the cooled stream (5) exiting the second heat exchanger (7) to the reboiler (15),
- at least a fourth conduit configured to send the cooled stream (5) exiting the reboiler (15) to the distillation column (11),
- at least a fifth conduit configured to withdraw the vapor phase (17) from the top of the distillation column and to send it to the condenser (19),
- at least a sixth conduit configured to return the liquid (23) enriched in methane formed in the condenser (19) to the distillation column (11),
- at least a seventh conduit configured to send the nitrogen vapor (9) exiting the condenser (19) to the first heat exchanger (3), and
- at least an eighth conduit configured to withdraw the liquid (13) containing at least 99.5 mol%, preferably at least 99.9 mol%, of methane from the bottom of the column as a final product.

10. The apparatus (100) according to claim 9, comprising a third (25) heat exchanger immersed in a bath of liquid nitrogen (21) configured to cool the liquid (13) withdrawn from the bottom of the distillation column and to vaporize the liquid nitrogen (21) to produce the nitrogen vapor (9), and at least a ninth conduit configured to send the produced nitrogen vapor (9) to the first heat exchanger (3).

11. The apparatus (100) according to claim 9 or 10, comprising a storage (29) of liquid nitrogen connected to the second heat exchanger (7) and/or the third heat exchanger (25) and/or the condenser (19).
